(19) Europäisches Patentamt
European Patent Office
Office européen des brevets

(11) Publication number : 0 381 318 B1

(12) # EUROPEAN PATENT SPECIFICATION

(45) Date of publication of patent specification : **20.10.93 Bulletin 93/42**

(51) Int. Cl.$^5$ : **A61K 7/06**

(21) Application number : **90300262.4**

(22) Date of filing : **10.01.90**

(54) **Hair-conditioning composition.**

(30) Priority : **01.02.89 GB 8902179**

(43) Date of publication of application : **08.08.90 Bulletin 90/32**

(45) Publication of the grant of the patent : **20.10.93 Bulletin 93/42**

(84) Designated Contracting States : **DE FR GB IT**

(56) References cited :
**EP-A- 0 298 402**
**US-A- 3 562 786**

(73) Proprietor : **Dow Corning Limited
Inveresk House 1 Aldywch
London WC2R 0HF (GB)**

(72) Inventor : **Donkers, Annemieke Constantia Maria
Le Cap Vert Boulevard de la Taverniere
Les Vigies (ap120) F-06210 Mandelieu (FR)**
Inventor : **Wright, Shirley Elizabeth
Avenue Leon Jourez 57 4 eme Etage
B-1420 Briane l'Alleud (BE)**

(74) Representative : **Vandamme, Luc Johan Roger
Dow Corning Limited Cardiff Road
Barry South Glamorgan CF6 7YL Wales (GB)**

**EP 0 381 318 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with conditioning compositions and is particularly concerned with conditioning compositions for human hair.

It has become a common practice to wash human hair frequently in order to clean it and it is normal to use for this purpose a shampoo containing a synthetic detergent. There are many shampoo formulations available which are effective to clean the hair, but generally the detergents employed have a tendency to leave the hair in a condition such that it is difficult to comb when wet and somewhat unmanageable when dried. It has therefore also become a practice to apply to the hair conditioner materials intended to improve the properties of the shampooed hair. These materials may be applied to the hair in admixture with the shampoo or applied to the wet shampooed hair as a separate conditioner, for example as a rinse.

Various proposals have been made concerning the formulation of compositions intended for use, for example on human hair with a view to conditioning the hair, i.e. imparting to the hair one or more desirable properties, for example ease of combing or detangling of wet hair, or ease of combing, lustre, a fullness or body accompanied by a certain liveliness, manageability and avoidance of static in the dried hair. Such compositions may take the form for example of shampoos, conditioners or rinses and all such compositions are referred to herein as conditioning compositions. It is a common practice to employ cationic surfactants for example organic quaternary ammonium compounds such as stearyldimethylbenzylammonium chloride, cetyltrimethylammonium bromide and dimethylbis(hydrogenated tallow)ammonium chloride, for their hair conditioning properties. These materials are effective in many respects, but in order to provide some of the desired properties it is necessary to employ significant amounts of the surfactant and as a consequence other properties are impaired. This in turn has led to proposals to include other selected ingredients in conditioning compositions to impart softness and other properties to the hair. It has been proposed for example, to employ certain polydiorganosiloxanes to impart increased softness to the hair. Other proposals have been made to employ in hair treating compositions certain organosilicon compounds having quaternary ammonium salt groups. For example, hair treating compositions comprising certain silanes or polysiloxanes having quaternary ammonium groups in combination with polysiloxanes having certain amino groups are described and claimed in G.B. Patent Specification 2 144 329. These compositions are effective to provide a variety of desirable properties to shampooed hair but it is recommended that they are not used frequently on the hair due to a tendency of certain polysiloxanes having amino groups to adhere strongly to the hair for a considerable time, and to resist removal during normal shampooing. Also the polysiloxanes having siloxane units including quaternary ammonium salt groups generally require a series of preparation steps and their manufacture can be costly.

We have now found that conditioning compositions containing certain polydiorganosiloxane-polyoxyalkylene copolymers demonstrate good conditioning effects on human hair.

The present invention provides in one of its aspects the use of a crosslinked polydiorganosiloxanepolyoxyalkylene copolymer comprising siloxane units (i) $R_aR'SiO_{\frac{3-a}{2}}$, (ii) $R_bSiO_{\frac{4-b}{2}}$ and at least one bridging unit (iii) $R_aSiO_{\frac{3-a}{2}}.X.O_{\frac{3-a}{2}}SiR_a$ wherein each R represents a monovalent hydrocarbon group, each R' represents a polyoxyalkylene group linked to the silicon atom through a divalent hydrocarbon linkage, each X represents a divalent organic or organosiloxane group linked to the two silicon atoms of the or each bridging unit (iii) via a Si-C linkage, a has the value 0, 1 or 2 and b has the value 0, 1, 2 or 3 in the formulation of a conditioning composition in the form of an emulsion.

According to another aspect of the invention there are provided conditioning compositions in the form of an emulsion comprising the above-mentioned crosslinked polydiorganosiloxane-polyoxyalkylene copolymers and being in oil-in-water form and/or comprising additionally detersive anionic surfactants.

Polydiorganosiloxane-polyoxyalkylene copolymers suitable for use in the present invention have been described in detail in European Patent application 0 298 402. They include those having the partial general formula

$$R_fR'_gSiO(\underset{\underset{|}{X}}{\underset{|}{R}}SiO)_c(RR'SiO)_y(R_2SiO)_zSiR_fR'_g$$

wherein c has the value 1, 2, 3, 4 or 5, y has a value from 1 to 10, z has a value from 0 to 700 and the value of y + z is not less than 40, the sum of g and f being 3 and in which X is further linked to a similar polydiorganosiloxane-polyoxyalkeylene copolymer via its other valency. In preferred copolymers c has the value 1. In those cases where c has a greater value each linkage X may provide a bridge between the same polysiloxane

chains or may make a bridge with another polysiloxane chain.

Preferred copolymers are those in which each monovalent hydrocarbon group R is, independently, a saturated or unsaturated aliphatic group having up to 25 carbon atoms for example a methyl, ethyl, propyl, hexyl, decyl, dodecyl, octadecyl or vinyl group, or an aromatic group for example phenyl. Especially preferred are those in which a significant proportion of the groups R are aliphatic groups having 10 to 20 carbon atoms. Preferably at least 50% of the siloxane units of the general formula (ii) of the polydiorganosiloxane-polyoxyalkylene copolymer have a silicon-bonded aliphatic substituent R which has from 10 to 20 carbon atoms. Most preferably at least 80% of the siloxane units of the general formula (ii) have such silicon-bonded substituent. Generally such siloxane units will only have one such aliphatic substituent, any other preferably being a lower alkyl group, for example methyl. In the most preferred copolymers the larger aliphatic R groups have 12 to 16 carbon atoms

Each group R' is an oxyalkylene group linked to the silicon atom through a divalent hydrocarbon linkage R" which may be an alkylene group having for example 2 or 3 carbon atoms. Each group R' may be of the formula $R''(OCH_2CH_2)_d(OCHCH_3CH_2)_eOZ$ wherein Z represents a terminal group which is not adversely reactive with other ingredients of the conditioner composition and does not interfere with preparation of the polydiorganosiloxane-polyoxyalkylene copolymer for example an acyl group, e.g. acetyl, propionyl, butyryl, isobutyryl, lauroyl, myristoyl, stearoyl, 3-carboxypentadecanoyl, an alkyl group e.g. methyl, ethyl, propyl, butyl, decyl, siloxanyl group e.g. $-Si(CH_3)_2O(CH_3)_2Si-$, or a hydrogen atom. In the preferred polyoxyalkylene chains d has a value from 4 to 40 and e has a value of up to 40, at least 50% of the oxyalkylene groups being oxyethylene groups ($OCH_2CH_2$). Whilst the groups R' may be present in the terminal units of the polysiloxane chains it is preferred that g has the value 0 and f has the value 3.

Each group X is a divalent organic or organosiloxane group linked to siloxane units of two polysiloxane chains via a Si-C group and contains no bonds hydrolysable in the conditioner composition or under normal conditions of exposure of hair treated therewith. Preferred groups X are according to the formula $CH_2CHR'''R''''CHR'''CH_2$ in which R''' represents a hydrogen atom or an aliphatic group having up to 3 carbon atoms and R'''' represents a divalent group, for example an alkylene group having 1 to 10 carbon atoms e.g. methylene, dimethylene, trimethylene, pentamethylene, a cycloalkylene group e.g. cyclohexylene, a divalent aromatic group e.g. p-phenylene or o-phenylene, an oxygen containing group e.g. $-COOCH_2CH_2OOC-$ and $-CH_2OCH_2-$ or a group of the formula $-SiR_2OSiR_2$.

The crosslinked polydiorganosiloxane-polyoxyalkylene copolymers used in the present invention are crosslinked sufficiently to provide a network of polydiorganosiloxane chains soluble in, or at least readily dispersible in, a non-polar liquid. These copolymers tend to be insoluble in water. They are not sufficiently crosslinked to cause the material to gel. They may be produced by the $\alpha$-olefin addition of vinyl containing components corresponding to the crosslinking material and the polyoxyalkylene group (and optionally an aliphatic group R) to a siloxane polymer backbone which may have, for example, the general formula

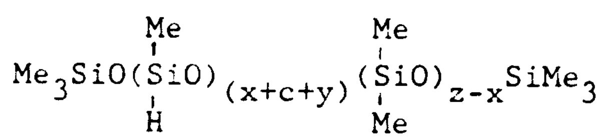

where x = 0 to 100, c = 1 to 5, y = 1 to 10, z = 0 to 600 and Me represents the methyl group. For example, where a 12 carbon aliphatic radical R is desired 1-dodecene may be used as the radical precursor in the reaction. The vinyl terminated crosslinking compound may be for example tetramethyldivinyldisiloxane of the formula

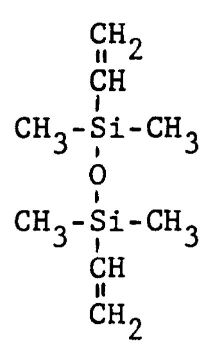

The polyoxyalkylene is similarly vinyl terminated at one end. A portion of the aliphatic radical precursor may he added to the siloxane polymer backbone material along with a suitable hydrosilylation catalyst under a nitrogen atmosphere. The divinyl crosslinking agent may be reacted next, again in the presence of an appropriate hydrosilylation catalyst and heat. Then the vinyl polyoxyalkylene may be added to the reaction vessel and reacted using an appropriate catalyst and heat. Finally, the remainder of the aliphatic radical precursor may be added to the reaction vessel and reacted with the remaining siloxane hydride reaction sites.

Compositions according to the invention are in the form of emulsions. Preferred are the oil-in-water emulsions but water-in-oil emulsions are also possible. Conditioning compositions according to the invention may be formulated as pure conditioners or they may be formulated as shampoo conditioners, in which case they will be oil-in-water emulsions.

Compositions according to the invention may also contain other materials, for example surfactants, thickeners, alcohols and silicone components. The extra components will differ according to the specific type of composition which one desires to produce. For example an oil-in-water conditioning composition generally comprises apart from the polydiorganosiloxane-polyoxyalkylene copolymer and water, an alcohol which forms the oil phase and a thickener or quaternary ammonium salt. A water-in-oil conditioning composition will comprise for example as extra ingredients a silicone oil, preferably a volatile silicone oil such as cyclomethicone and a salt to stabilise the emulsion. A shampoo conditioning composition will comprise as extra ingredient a detersive, preferably an anionic surfactant. Examples of the extra ingredients include quaternary ammonium salts, silicones, surfactants such as aliphatic fatty amines and their derivatives e.g. octadecylamine acetate and quaternary ammonium halides, sulphonic acids e.g. dodecyl benzene sulphonic acid, ethylene oxide adducts of octyl or nonyl phenols e.g. octylphenoxypolyethoxyethanol, condensation products of aliphatic alcohols and ethylene oxide, monoesters of alcohols and fatty acids e.g. sodium lauryl sulphate glyceryl stearate, sorbitan monolaurate and polyoxythylenemonostearate, and polyvinyl alcohol. The surfactant component is generally employed in amounts of about 0.01% to about 5% by weight of the composition in the case of pure conditioning compositions, but this may go up to 40% in the case of shampoo conditioning compositions.

If desired, the composition may include other ingredients as stabilisers, for example non-ionic surfactants as aforesaid, thickeners e.g. carboxymethyl cellulose, hydroxypropyl cellulose and guar gum, colorants, perfumes, bactericides, solvents, preservatives, and conventional hair conditioning ingredients e.g. handle modifiers e.g. fatty acid salts esters and ethers and long chain alkyl alcohols, waxes, oils and organic quaternary compounds.

When the composition is formulated as a shampoo suitable sequestering agents and detergents for the cleaning of hair are included, for example those based on fatty alcoholethoxylates. Examples of such detergents are sodium lauryl sulphate, sodium lauryl ether sulphate, ammonium lauryl sulphate and triethanolamine lauryl sulphate. The surfactant chosen for the emulsification of the polysiloxane may in some cases function also as the detergent component of the shampoo formulation. When so formulated, the proportion of the surfactant used will normally be much larger than the amount used to provide the stability of the emulsion. In the case of shampoo conditioning compositions it is preferred to combine a polydiorganosiloxane-polyoxyalkylene copolymer and a detersive surfactant which are compatible in such a way that the surfactant tends to solubilise the polydiorganosiloxane-polyoxyalkylene copolymer. Such solubilised systems are included in the term emulsion where used in this specification. This is generally achieved by adjusting the higher alkyl content and type of the polydiorganosiloxane-polyoxyalkylene copolymer to be the same or closely similar in carbon content to the higher alkyl type and content of the surfactant. For example if a lauryl sulphate surfactant is used in the composition it is preferred that at least a significant part of the R substituents in the polydiorganosiloxane-polyoxyalkylene copolymer are lauryl groups or at least alkyl groups with 10 to 14 carbon atoms. Similarly a surfactant having a $C_{16}$ alkyl group would preferably be used in combination with a polydiorganosiloxane-polyoxyalkylene copolymer in which a significant part of the R groups is an alkyl group with 14 to 18 carbon atoms, preferably 16 carbon atoms. This allows the shampoo conditioning composition to be formulated as a clear composition which was, up to now, only possible if water soluble polysiloxane-polyoxyalkylene copolymers were used. The present invention accordingly makes it possible to formulate clear shampoo compositions which provide better conditioning characteristics than was previously possible by using the crosslinked silicone copolymers which are water insoluble and which are hence believed to deposit more readily onto the hair.

Also included within the scope of the invention is a process for the treatment of hair which comprises applying thereto a composition according to the invention. The treatment is preferably done after the hair has been shampooed and the shampoo rinsed off, but it may be done simultaneously with the shampooing of the hair, the latter being more appropriate when a composition according to the invention is formulated as a shampoo.

Compositions according to the present invention allow the formulation of hair conditioning compositions as a shampoo or as a rinse for treating shampooed hair, using a minimal number of ingredients in comparatively

small quantities, still providing a desirable conditioning effect.

In order that the invention may become more clear there now follows a description of example conditioners which are illustrative of the invention. The symbol Me signifies the methyl group. All percentages are by weight unless specified otherwise.

Example 1

A crosslinked diorganosiloxane-polyoxyalkylene was prepared based on the following formulation

| | |
|---|---|
| 15.7% | $Me_3SiO(MeHSiO)_{41}SiMe_3$ |
| 36.2% | 1st dodecene addition |
| 15.0% | 2nd dodecene addition |
| 9-0% | Isostearyl alcohol |
| 23.6% | 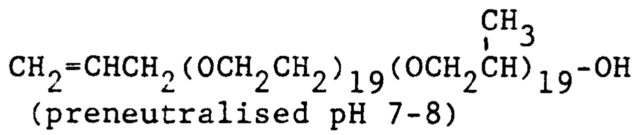 (preneutralised pH 7-8) |
| 0.5% | 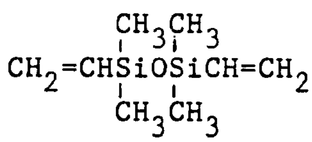 |
| 14 ppm | Pt (chloroplatinic acid (2% $H_2PtCl_6.6H_2O$)/IPA) hydrosilation catalyst |

As will be appreciated by those skilled in the art the siloxane backbone material includes a mixture in which the methyl siloxane hydride units may comprise anywhere from 30 to 60 units on a given siloxane molecule the mean being approximately 41 as indicated.

The dodecene is at 91% olefinic activity. The indicated percentages are by weight. The indicated pH of the vinyl polyoxyalkylene glycol was determined based on a 15% by weight solution thereof in water.

All of the siloxane backbone material and 10% of the first dodecene addition was loaded into the reactor with agitation and the pressure was reduced by about 25 millimetres of mercury for one minute, after which the vessel was backfilled with nitrogen. With a nitrogen sweep the mixture was heated to 85°C and 25% of the catalyst solution and the remainder of the first dodecene addition were added over a period of two to three hours holding the temperature at 105 to 115°C. After all of the first dodecene addition was completed the vessel was held at 110°C for 15 minutes.

The temperature was then dropped to 80°C and all of the divinyl tetramethylsiloxane crosslinking agent added along with an additional 25% of the catalyst solution. The vessel reaction temperature was returned to 110°C and held there for 15 minutes.

At this point the isostearyl alcohol was added along with the vinyl polyoxyalkylene. The vessel was again degassed and backfilled as in the first step described above.

Another 30% of the catalyst solution was added and the temperature increased to 110°C and held there for 30 minutes.

The remainder of the catalyst was then added to the vessel which was heated to 120°C and held there for 30 minutes. The second dodecene addition was then made and the vessel held at 120°C for an additional hour. Thereafter the composition was cooled and removed from the reaction vessel.

The resulting polyorganosiloxane-polyoxyalkylene copolymer had the general formula

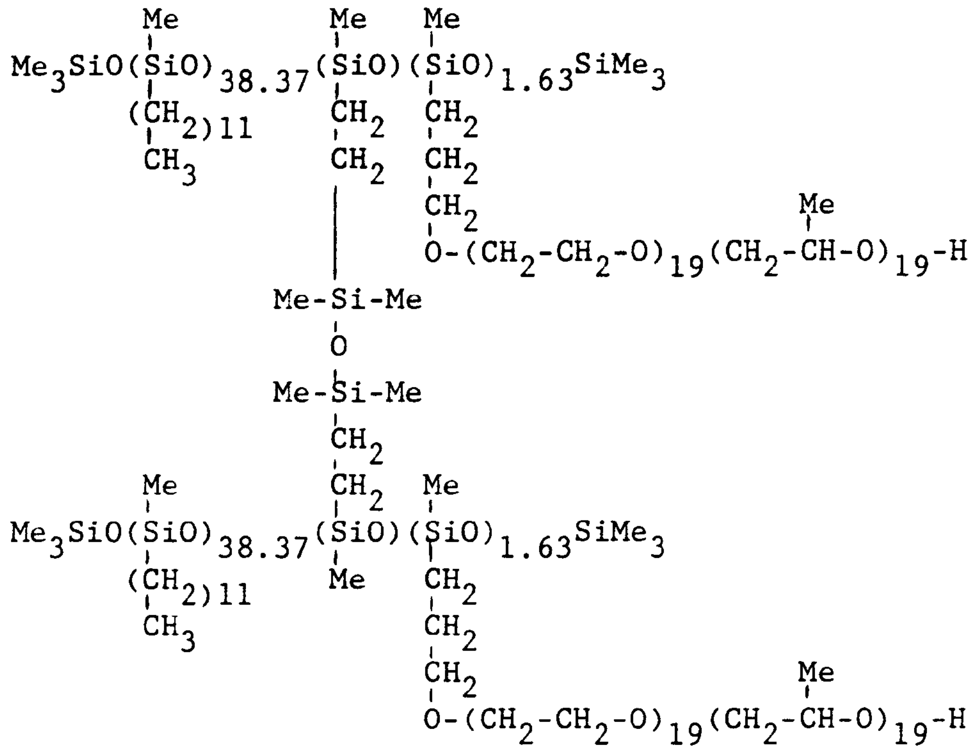

Thus the polydiorganosiloxane-polyoxyalkylene copolymer included no dimethyl siloxane groups but an average of 38.37 aliphatic added siloxane groups. The numerical values indicated were calculated based on starting ingredients. The divinyl disiloxane was added at only about 0.5% by weight creating one crosslink per polymer.

The polydiorganosiloxane-polyoxyalkylene copolymer was used to prepare sample hair shampoos according to the following formulation

| Ingredient | % by Weight |
|---|---|
| Copolymer | x |
| Linoleamide | y |
| Sodium laurylether sulphate | 30 |
| Coconut fatty acid diethanolamide | 2 |
| Polyethylene glycol-120 methyl glucose dioleate | 2.5 |
| Water (to 100%) | (65.5-x-y) |

The polydiorganosiloxane-polyoxyalkylene copolymer was dispersed in the linoleamide and added to a mixture of the sodium lauryl sulphate and water. The coconut amide was added to this mixture followed by the polyethylene glycol oleate and water. Various compositions were formulated with ratios of x:y of 1:1, 1:2, 1:3 using 0.5, 1.0, 1.5 and 2% by weight of the polydiorganosiloxane-polyoxyalkylene copolymer. 5g samples of these compositions were tested on hair and combing and handle were evaluated in the following way.

A hair swatch (European natural brown hair), was wetted and washed twice using a shampoo, based on 20% sodium lauryl ether sulphate and 3.5% of linoleic diethanolamide. The hair swatch was rinsed well in running water. Then the conditioning composition was applied to the swatch in a ratio of 5ml per 7.8g of hair. After 1 minute, the hair was combed in order to obtain an equal spreading of the conditioner, and the hair swatch was rinsed for 15 seconds. When the excess of water had been removed, the hair swatch was tested for wet combability. This was done by passing a fine plastic comb through the hair, and assessing the ease of combing. The hair swatch was then dried during one hour in an air-circulating oven at 70°C. When the hair was cooled

to room temperature dry combability was assessed. The handle was assessed by feeling the hair and considering its softness, silkiness and body. This test was carried out by a panel of experienced people.

When comparing swatches treated with these conditioning compositions according to the invention with a Standard swatch, which was only shampooed, without any conditioner having been applied a conditioning effect was observed. Wet combing was better with increased proportions of the polydiorganosiloxane-oxyalkylene copolymer, the least drag being at 2.0%. Dry combing was good with all the compositions. The hair had soft, silky, lively handle and there was an improved gloss/lustre to the hair with all of the example compositions.

Example 2

The following clear shampoo conditioning compositions were prepared:

| Ingredient | % by Weight |
|---|---|
| Copolymer of Example 1 | 3 |
| Sodium laurylether sulphate | 9 |
| Coconut fatty acid diethanolamide | 3 |
| Polyethylene glycol-120 methyl glucose dioleate | 3 |
| Sodium Chloride | 1 |
| Citric acid solution (50%) | qs |
| Water | up to 100% |

and

| Ingredient | % by Weight |
|---|---|
| Copolymer of Example 1 | 3 |
| Sodium laurylether sulphate | 9 |
| Talloweth-60 myristyl glycol | 3.5 |
| Sodium Chloride | 3.2 |
| Citric acid solution (50%) | qs |
| Water | up to 100% |

The polydiorganosiloxane-polyoxyalkylene copolymer was added to the sodium lauryl sulphate and heated till it became almost clear. The other ingredients were added to this mixture followed by heating and agitation at 60°C until the mixture was homogeneous. When cooled the pH was adjusted with the citric acid.

A hair swatch (European natural brown hair) was wetted and washed twice using the shampoo conditioning compositions of the example. The hair swatch was rinsed well in running water. When the excess water was removed the hair swatch was tested as in Example 1. When comparing swatches treated with these conditioning composition according to the invention, with a swatch treated with a shampoo conditionining composition similar to the second composition of Example 2, but leaving out the polydiorganosiloxane-polyoxyalkylene copolymer, an improved conditioning effect was observed as well as improvements in wet combing, dry combing, handle and gloss/lustre.

Example 3

The following conditioning composition was prepared:

| Ingredient | % by Weight |
|---|---|
| Copolymer of Example 1 | 2 |
| Hydroxyethylcellulose 250 | 1 |
| Cetyl alcohol | 3 |
| Ceteareth 20 | 1.5 |
| Water | up to 100% |

The polydiorganosiloxane-polyoxyalkylene copolymer was added to the ceateareth and heated till it became almost clear. The other ingredients were dispersed in the water at 72°C to which, after cooling to 50°C, the first mixture was added under stirring.

A hair swatch (European natural brown hair) was wetted and washed twice using a shampoo composition as described in Example 1. The hair swatch was rinsed well in running water. Then the conditioning composition was applied to the swatch in a ratio of 5ml per 7.8g of hair. After 1 minute the hair was combed in order to obtain an equal spreading of the conditioner and the hair swatch rinsed for 15 seconds. When the excess water was removed the hair swatch was tested as in Example 1. When comparing swatches treated with this conditioning composition according to the invention with a swatch treated with a conditioning composition similar to the composition of Example 3, but leaving out the polydiorganosiloxane-polyoxyethylene copolymer, an improved conditioning effect was observed as well as improvements in wet combing, dry combing, handle and gloss/lustre.

The conditioning effects were also compared with a composition which was identical to the ones used in this example except that the polydiorganosiloxane-polyoxyethylene copolymer of Example 1 was replaced with a polydiorganosiloxane-polyoxyethylene copolymer which does not have any crosslinking groups in it. The conditioning effect of this formulation was markedly inferior to the one of the Example composition described above.

Example 4

The following conditioning composition was prepared:

| Ingredient | % by Weight |
|---|---|
| Copolymer of Example 1 | 3 |
| Cyclomethicone | 32 |
| Sodium Chloride | 2 |
| Water | up to 100% |

The polydiorganosiloxane-polyoxyethylene copolymer was added to the cyclomethicone and the water with the sodium chloride dissolved in it, was added slowly under high shear to give a water-in-oil emulsion.

A hair swatch (European natural brown hair) was wetted and washed twice using a shampoo composition as described in Example 1. The hair swatch was rinsed well in running water. Then the conditioning composition was applied to the swatch in a ratio of 5ml per 7.8g of hair. After 1 minute the hair was combed in order to obtain an equal spreading of the conditioner and the hair swatch rinsed for 15 seconds. When the excess water was removed the hair swatch was tested as in Example 1. When comparing swatches treated with this conditioning composition according to the invention with a swatch treated with a conditioning composition similar to the composition of Example 3, but leaving out the polydiorganosiloxane-polyoxyethylene copolymer, an improved conditioning effect was observed as well as improvements in wet combing, dry combing, handle and gloss/lustre.

## Claims

1. The use of a crosslinked polydiorganosiloxane-polyoxyalkylene copolymer comprising siloxane units (i)

$R_aR'SiO_{\frac{3-a}{2}}$, (ii) $R_bSiO_{\frac{4-b}{2}}$ and at least one bridging unit (iii) $R_aSiO_{\frac{3-a}{2}}.X.O_{\frac{3-a}{2}}SiR_a$ wherein each R represents a monovalent hydrocarbon group, each R' represents a polyoxyalkylene group linked to the silicon atom through a divalent hydrocarbon linkage, each X represents a divalent organic or organosiloxane group linked to the two silicon atoms of the or each bridging unit (iii) via a Si-C linkage, a has the value 0, 1 or 2 and b has the value 0, 1, 2 or 3 in the formulation of a hair-conditioning composition in the form of an emulsion.

2. The use of a crosslinked copolymer according to Claim 1 in the formulation of a hair-conditioning composition in the form of an oil-in-water emulsion.

3. A conditioning composition in the form of an oil-in-water emulsion comprising a silicone compound characterised in that said compound is a crosslinked polydiorganosiloxane-polyoxyalkylene copolymer comprising siloxane units (i) $R_aR'SiO_{\frac{3-a}{2}}$, (ii) $R_bSiO_{\frac{4-b}{2}}$ and at least one bridging unit (iii) $R_aSiO_{\frac{3-a}{2}}.X.O_{\frac{3-a}{2}}SiR_a$ wherein each R represents a monovalent hydrocarbon group, each R' represents a polyoxyalkylene group linked to the silicon atom through a divalent hydrocarbon linkage, each X represents a divalent organic or organosiloxane group linked to the two silicon atoms of the or each bridging unit (iii) via a Si-C linkage, a has the value 0, 1 or 2 and b has the value 0, 1, 2 or 3.

4. A conditioning composition in the form of an emulsion comprising a silicone compound and detersive anionic surfactants characterised in that said compound is a crosslinked polydiorganosiloxane-polyoxyalkylene copolymer comprising siloxane units (i) $R_aR'SiO_{\frac{3-a}{2}}$, (ii) $R_bSiO_{\frac{4-b}{2}}$ and at least one bridging unit (iii) $R_aSiO_{\frac{3-a}{2}}.X.O_{\frac{3-a}{2}}SiR_a$ wherein each R represents a monovalent hydrocarbon group, each R' represents a polyoxyalkylene group linked to the silicon atom through a divalent hydrocarbon linkage, each X represents a divalent organic or organosiloxane group linked to the two silicon atoms of the or each bridging unit (iii) via a Si-C linkage, a has the value 0, 1 or 2 and b has the value 0, 1, 2 or 3.

5. A conditioning composition according to either Claim 3 or Claim 4 characterised in that at least 50% of all the siloxane units (ii) have a R substituent which is an aliphatic group having from 10 to 20 carbon atoms.

6. A conditioning composition according to any one of Claims 3 to 5 characterised in that the aliphatic group has from 12 to 16 carbon atoms.

7. A conditioning composition according to any one of Claims 3 to 6 characterised in that each polyoxyalkylene group consists of from 4 to 40 oxyethylene groups and from 4 to 40 oxypropylene groups, there being at least 50% oxyethylene groups.

8. A conditioning composition according to any one of Claims 3 to 7 characterised in that each group X is a divalent organic or organosiloxane group according to the formula $CH_2CHR'''R''''CHR'''CH_2$ in which R''' represents a hydrogen atom or an aliphatic group having up to 3 carbon atoms and R'''' represents an alkylene group having 1 to 10 carbon atoms, a cycloalkylene group, a divalent aromatic group, an oxygen containing divalent group or a group of the formula $-SiR_2OSiR_2-$.

9. A shampoo conditioning composition according to Claim 4, or any claim dependent therefrom, characterised in that it is optically clear.

10. A process for treating hair which comprises applying to the hair a conditioning composition characterised in that the composition is according to any one of the preceding claims.

**Patentansprüche**

1. Verwendung eines vernetzten Polydiorganosiloxan-Polyoxyalkylen-Copolymers umfassend Siloxaneinheiten (i) $R_aR'SiO_{\frac{3-a}{2}}$, (ii) $R_bSiO_{\frac{4-b}{2}}$ und mindestens eine verbrückende Einheit (iii) $R_aSiO_{\frac{3-a}{2}}X{\cdot}O_{\frac{3-a}{2}}SiR_a$, worin jeder Rest R eine monovalente Kohlenwasserstoffgruppe bedeutet, jeder Rest R' eine Polyoxyalkylengruppe, die an ein Siliciumatom über eine divalente Kohlenwasserstoffbindung gebunden ist, bedeutet, jeder Rest X eine divalente organische oder Organosiloxangruppe, die an zwei Siliciumatome der oder jeder verbrückenden Einheit (iii) über eine Si-C-Bindung gebunden ist, bedeutet, a den Wert

0, 1 oder 2 hat und b den Wert 0, 1, 2 oder 3 hat, für die Formulierung einer haarkonditionierenden Zusammensetzung in Form einer Emulsion.

2. Verwendung eines vernetzten Copolymers nach Anspruch 1 für eine Formulierung einer haarkonditionierenden Zusammensetzung in Form einer Öl-in-Wasser-Emulsion.

3. Konditionierzusammensetzung in Form einer Öl-in-Wasser-Emulsion umfassend eine Silikonverbindung, dadurch gekennzeichnet, daß die Verbindung ein vernetztes Polydiorganosiloxan-Polyoxyalkylen-Copolymer ist, das Siloxaneinheiten (i) $R_aR'SiO_{\frac{3-a}{2}}$, (ii) $R_bSiO_{\frac{4-b}{2}}$ und mindestens eine verbrückende Einheit (iii) $R_aSiO_{\frac{3-a}{2}}.X{\cdot}O_{\frac{3-a}{2}}SiR_a$, worin jeder Rest R eine monovalente Kohlenwasserstoffgruppe bedeutet, jeder Rest R' eine Polyoxyalkylengruppe, die an ein Siliciumatom über eine divalente Kohlenwasserstoffbindung gebunden ist, bedeutet, jeder Rest X eine divalente organische oder Organosiloxangruppe, die an zwei Siliciumatome der oder jeder verbrückenden Einheit (iii) über eine Si-C-Bindung gebunden ist, bedeutet, a den Wert 0, 1 oder 2 hat und b den Wert 0, 1, 2 oder 3 hat, umfaßt.

4. Konditionierzusammensetzung in Form einer Emulsion umfassend eine Silikonverbindung und reinigende anionische Tenside, dadurch gekennzeichnet, daß die Verbindung ein vernetztes Polydiorganosiloxan-Polyoxyalkylen-Copolymer ist, das Siloxaneinheiten (i) $R_aR'SiO_{\frac{3-a}{2}}$, (ii) $R_bSiO_{\frac{4-b}{2}}$ und mindestens eine verbrückende Einheit (iii) $R_aSiO_{\frac{3-a}{2}}.X{\cdot}O_{\frac{3-a}{2}}SiR_a$ , worin jeder Rest R eine monovalente Kohlenwasserstoffgruppe bedeutet, jeder Rest R' eine Polyoxyalkylengruppe, die an ein Siliciumatom über eine divalente Kohlenwasserstoffbindung gebunden ist, bedeutet, jeder Rest X eine divalente organische oder Organosiloxangruppe, die an zwei Siliciumatome der oder jeder verbrückenden Einheit (iii) über eine Si-C-Bindung gebunden ist, bedeutet, a den Wert 0, 1 oder 2 hat und b den Wert 0, 1, 2 oder 3 hat, umfaßt.

5. Konditionierzusammensetzung nach Anspruch 3 oder Anspruch 4, dadurch gekennzeichnet, daß mindestens 50% aller Siloxaneinheiten (ii) einen Substituenten R haben, der eine aliphatische Gruppe mit 10 bis 20 Kohlenstoffatomen ist.

6. Konditionierzusammensetzung nach einem der Ansprüche 3 bis 5, dadurch gekennzeichnet, daß die aliphatische Gruppe 12 bis 16 Kohlenstoffatome aufweist.

7. Konditionierzusammensetzung nach einem der Ansprüche 3 bis 6, dadurch gekennzeichnet, daß jede Polyoxyalkylengrupe aus 4 bis 40 Oxyethylengruppen und 4 bis 40 Oxypropylengruppen besteht, wobei mindestens 50% Oxyethylengruppen vorhanden sind.

8. Konditionierzusammensetzung nach einem der Ansprüche 3 bis 7, dadurch gekennzeichnet, daß jede Gruppe X eine divalente organische oder Organosiloxangruppe ist mit der Formel $CH_2CHR'''R''''CHR'''CH_2$, worin R''' ein Wasserstoffatom oder eine aliphatische Gruppe mit bis zu 3 Kohlenstoffatomen bedeutet und R'''' eine Alkylengruppe mit 1 bis 10 Kohlenstoffatomen, eine Cycloalkylengruppe, eine divalente aromatische Gruppe, eine Sauerstoff enthaltende divalente Gruppe oder eine Gruppe der Formel $-SiR_2OSiR_2-$ bedeutet.

9. Shampookonditionierzusammensetzung nach Anspruch 4 oder einem davon abhängigen Anspruch, dadurch gekennzeichnet, daß sie optisch klar ist.

10. Verfahren zur Behandlung von Haar, umfassend, daß man auf das Haar eine Konditionierzusammensetzung aufbringt, dadurch gekennzeichnet, daß die Zusammensetzung eine gemäß einem der vorhergehenden Ansprüche ist.

## Revendications

1. L'utilisation d'un copolymère réticulé de polydiorganosiloxane-polyoxyalkylène, constitué d'unités siloxanes (i) $R_aR'SiO_{\frac{3-a}{2}}$, (ii) $R_bSiO_{\frac{4-b}{2}}$ et d'au moins une unité de pontage (iii) $R_aSiO_{\frac{3-a}{2}}.X.O_{\frac{3-a}{2}}SiR_a$ où chaque R représente un groupe hydrocarboné monovalent, chaque R' représente un groupe polyoxyalkylène lié à l'atome de silicium par un chaînon hydrocarboné divalent, chaque X représente un groupe organique ou organosiloxane lié aux deux atomes de silicium de l'unité de pontage ou de chaque unité de pontage

(iii) par une liaison Si-C, a a la valeur 0, 1 ou 2 et b a la valeur 0, 1, 2 ou 3, dans la formulation d'une composition de traitement capillaire sous forme d'émulsion.

**2.** L'utilisation d'un copolymère réticulé selon la revendication 1 dans la formulation d'une composition de traitement capillaire sous forme d'une émulsion huile-dans-l'eau.

**3.** Une composition de traitement sous forme d'une émulsion huile-dans-l'eau, constituée d'un composé silicone, caractérisée par le fait que ledit composé est un copolymère réticulé de polydiorganosiloxane-polyoxyalkylène constitué d'unités siloxanes (i) $R_aR'SiO_{\frac{3-a}{2}}$, (ii) $R_bSiO_{\frac{4-b}{2}}$ et d'au moins une unité de pontage (iii) $R_aSiO_{\frac{3-a}{2}}.X.O_{\frac{3-a}{2}}SiR_a$ où chaque R représente un groupe hydrocarboné monovalent, chaque R' représente un groupe polyoxyalkylène lié à l'atome de silicium par un chaînon hydrocarboné divalent, chaque X représente un groupe organique ou organosiloxane lié aux deux atomes de silicium de l'unité de pontage ou de chaque unité de pontage (iii) par une liaison Si-C, a a la valeur 0, 1 ou 2 et b a la valeur 0, 1, 2 ou 3.

**4.** Une composition de traitement sous forme d'une émulsion constituée d'un composé silicone et d'agents tensioactifs détergents anioniques, caractérisée par le fait que ledit composé est un copolymère réticulé de polydiorganosiloxane-polyoxyalkylène constitué d'unités siloxanes (i) $R_aR'SiO_{\frac{3-a}{2}}$, (ii) $R_bSiO_{\frac{4-b}{2}}$ et d'au moins une unité de pontage (iii) $R_aSiO_{\frac{3-a}{2}}.X.O_{\frac{3-a}{2}}SiR_a$ où chaque R représente un groupe hydrocarboné monovalent, chaque R' représente un groupe polyoxyalkylène lié à l'atome de silicium par un chaînon hydrocarboné divalent, chaque X représente un groupe organique ou organosiloxane lié aux deux atomes de silicium de l'unité de pontage ou de chaque unité de pontage (iii) par une liaison Si-C, a a la valeur 0, 1 ou 2 et b a la valeur 0, 1, 2 ou 3.

**5.** Une composition de traitement selon la revendication 3 ou la revendication 4, caractérisée par le fait qu'au moins 50 % de toutes les unités siloxanes (ii) ont un substituant R qui est un groupe aliphatique possédant de 10 à 20 atomes de carbone.

**6.** Une composition de traitement selon l'une quelconque des revendications 3 à 5, caractérisée par le fait que le groupe aliphatique possède de 12 à 16 atomes de carbone.

**7.** Une composition de traitement selon l'une quelconque des revendications 3 à 6, caractérisée par le fait que chaque groupe polyoxyalkylène est constitué de 4 à 40 groupes oxyéthylène et de 4 à 40 groupes oxypropylène, étant entendu qu'il y a au moins 50 % de groupes oxyéthylène.

**8.** Une composition de traitement selon l'une quelconque des revendications 3 à 7, caractérisée par le fait que chaque groupe X est un groupe organique ou organosiloxane divalent répondant à la formule $CH_2$ CHR'''R''''CHR'''$CH_2$, où R''' représente un atome d'hydrogène ou un groupe aliphatique possédant jusqu'à 3 atomes de carbone et R'''' représente un groupe alkylène possédant de 1 à 10 atomes de carbone, un groupe cycloalkylène, un groupe aromatique divalent, un groupe divalent contenant de l'oxygène, ou un groupe de formule $-SiR_2OSiR_2-$.

**9.** Une composition de shampoing selon la revendication 4 ou l'une quelconque des revendications qui en dépendent, caractérisée par le fait qu'elle est optiquement transparente.

**10.** Un procédé de traitement des cheveux qui consiste à leur appliquer une composition de traitement caractérisé par le fait que la composition est conforme à l'une quelconque des revendications qui précèdent.